(19) Europäisches Patentamt
European Patent Office
Office européen des brevets

(11) Veröffentlichungsnummer : **0 398 059 B1**

# (12) EUROPÄISCHE PATENTSCHRIFT

(45) Veröffentlichungstag der Patentschrift :
**25.08.93 Patentblatt 93/34**

(51) Int. Cl.$^5$ : **C07C 233/61,** C07D 207/16, A01N 53/00, A01N 43/36

(21) Anmeldenummer : **90108151.3**

(22) Anmeldetag : **28.04.90**

(54) **Cyclopropanoylaminosäureamid-Derivate.**

Die Akte enthält technische Angaben, die nach dem Eingang der Anmeldung eingereicht wurden und die nicht in dieser Patentschrift enthalten sind.

(30) Priorität : **13.05.89 DE 3915756**

(43) Veröffentlichungstag der Anmeldung :
**22.11.90 Patentblatt 90/47**

(45) Bekanntmachung des Hinweises auf die Patenterteilung :
**25.08.93 Patentblatt 93/34**

(84) Benannte Vertragsstaaten :
**BE CH DE FR GB IT LI NL**

(56) Entgegenhaltungen :
**EP-A- 0 170 842**
**DE-A- 2 515 113**
**FR-A- 2 139 837**
**GB-A- 2 072 165**
**US-A- 4 460 603**

(73) Patentinhaber : **BAYER AG**
**D-51368 Leverkusen (DE)**

(72) Erfinder : **Wollweber, Detlef, Dr.**
**Paul-Ehrlich-Strasse 17**
**D-5600 Wuppertal 1 (DE)**
Erfinder : **Hänssler, Gerd, Dr.**
**Am Arenzberg 58a**
**D-5090 Leverkusen 3 (DE)**

**EP 0 398 059 B1**

Anmerkung : Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Die vorliegende Erfindung betrifft neue Cyclopropanoylaminosäureamide, Verfahren zu deren Herstellung, sowie deren Verwendung als Schädlingsbekämpfungsmittel.

Die erfindungsgemäßen Substanzen besitzen eine ausgezeichnete Wirkung bei der Bekämpfung von Schädlingen. Inbesondere können die erfindungsgemäßen Substanzen als Fungizide, vor allem im Pflanzenschutz verwendet werden.

Gegenstand der vorliegenden Anmeldung sind somit Cyclopropanoylaminosäureamid-Derivate der allgemeinen Formel (I)

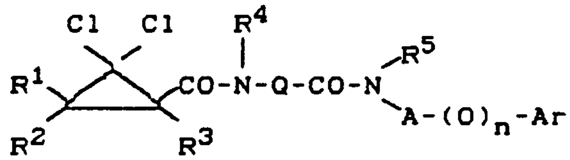

(I)

in welcher

| | |
|---|---|
| $R^1$, $R^2$, $R^3$, $R^4$ und $R^5$ | gleich oder verschieden sind und für Wasserstoff oder Alkyl stehen; |
| Q | für eine unsubstituierte oder substituierte, geradkettige oder verzweigte Alkylenkette steht, oder zusammen mit dem Rest $R^4$ und dem Stickstoffatom einen Pyrrolidinring bildet; |
| A | für eine geradkettige oder verzweigte Alkylenkette steht; |
| n | für eine Zahl 0 oder 1 steht und |
| Ar | für unsubstituiertes oder substituiertes Aryl steht. |

Weiterhin wurde gefunden, daß man die neuen Cyclopropanoylaminosäureamid-Derivate der Formel (I)

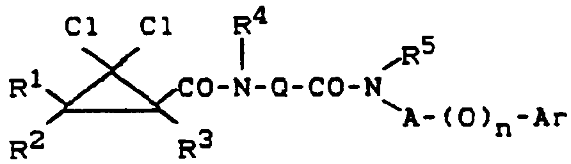

(I)

in welcher

| | |
|---|---|
| $R^1$, $R^2$, $R^3$, $R^4$ und $R^5$ | gleich oder verschieden sind und für Wassestoff oder Alkyl stehen; |
| Q | für eine unsubstituierte oder substituierte geradkettige oder verzweigte Alkylenkette steht, oder zusammen mit dem Rest $R^4$ und dem Stickstoffatom einen Pyrrolidinring bildet; |
| A | für eine geradkettige oder verzweigte Alkylenkette steht; |
| n | für eine Zahl 0 oder 1 steht und |
| Ar | für unsubstituiertes oder substituiertes Aryl steht |

erhält, wenn man

a) Cyclopropancarbonsäure der Formel (II)

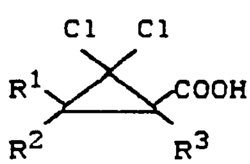

(II)

in welcher

$R^1$, $R^2$, und $R^3$ die oben angegebene Bedeutung haben bzw. deren carboxy-aktivierte Derivate, mit Aminosäure der Formel (III)

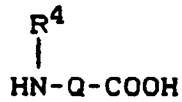

(III)

in welcher

$R^4$ und Q die oben angegebene Bedeutung haben, gegebenenfalls in Gegenwart eines Katalysators, gegebenenfalls in Gegenwart eines Säurebindemittels und gegebenenfalls in Gegenwart eines Verdünnungsmittels umsetzt, und die so erhaltenen Cyclopropanoylaminosäure der Formel (IV)

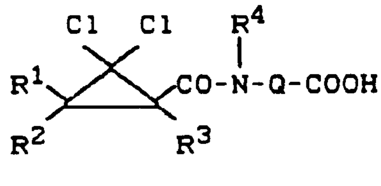

(IV)

in welcher

$R^1$, $R^2$, $R^3$, $R^4$ und Q die oben angegebene Bedeutung haben anschließend, gegebenenfalls nach Überführung in eine carboxy-aktivierte Form, mit einem Amin der Formel (V)

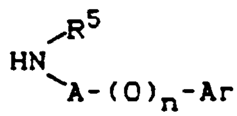

(V)

in welcher

$R^5$, A, n und Ar die oben angegebene Bedeutung haben, gegebenenfalls in Gegenwart eines Katalysators, gegebenenfalls in Gegenwart eines Säurebindemittels und gegebenenfalls in Gegenwart eines Verdünnungsmittels umsetzt,

oder

b) eine durch eine Aminoschutzgruppe geschützte Aminosäure der Formel (VI)

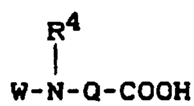

(VI)

in welcher $R^4$ und Q die oben angegebene Bedeutung haben und W für eine Aminoschutzgruppe steht, bzw. deren carboxy-aktivierte Derivate, gegebenenfalls in Gegenwart eines Katalysators, gegebenenfalls in Gegenwart eines Säurebindemittels und gegebenenfalls in Gegenwart eines Verdünnungsmittels mit einem Amin der Formel (V)

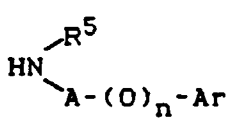

(V)

in welcher

$R^5$, A, n und Ar die oben angegebene Bedeutung haben, umsetzt und anschließend das so erhaltene Aminosäureamid der Formel (VII)

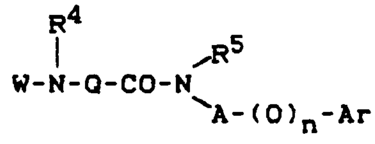 (VII)

in welcher
W, $R^4$, Q, $R^5$, A, n und Ar die oben angegebene Bedeutung haben, nach Abspaltung der Aminoschutzgruppe W mit Cyclopropancarbonsäure der Formel (II)

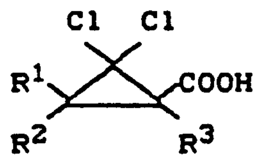 (II)

in welcher
$R^1$, $R^2$ und $R^3$ die oben angegebene Bedeutung haben, bzw. deren carboxy-aktivierten Derivaten, gegebenenfalls in Gegenwart eines Katalysator, gegebenenfalls in Gegenwart eines Säurebindemittels und gegebenenfalls in Gegenwart eines Verdünnungsmitels umsetzt.

Die Verbindungen der Formel (I) können ein oder mehrere Chiralitätszentren enthalten und somit in verschiedenen Enantiomeren- und Diastereomerengemischen vorliegen, die gegebenenfalls in üblicher Art und Weise getrennt werden können. Es werden sowohl die reinen Enantiomeren und Diastereomeren, als auch die Gemische erfindungsgemäß beansprucht.

Im folgenden wird der Einfachheit halber jedoch stets von Verbindungen der Formel (I) gesprochen, obwohl sowohl die reinen Verbindungen, als auch die Gemische mit unterschiedlichen Anteilen an enantiomeren und diastereomeren Verbindungen gemeint sind.

Die erfindungsgemäßen Verbindungen sind durch die Formel (I) allgemein definiert.

In den allgemeinen Formeln steht Alkyl, falls nicht anders definiert, für geradkettiges oder verzweigtes Alkyl mit vorzugsweise 1 bis 8, inbesondere 1 bis 6 und vor allem 1 bis 4 Kohlenstoffatomen, wobei Methyl, Ethyl, n- und i-Propyl, n-, i-, s- und t-Butyl beispielhaft und vorzugsweise genannt seien.

Die geradkettige oder verzweigte Alkylenkette in den Definitionen von Q enthalten vorzugsweise 1 bis 8, insbesondere 1 bis 6 und vor allem 1 bis 4 Kohlenstoffatome, wobei Methylen, 1,1- und 1,2-Ethylen, 1,1-, 1,2-, 1,3-, und 2,2-Propylen und 1,1-, 1,2-, 1,3-, 1,4-, 2,2-, 2,3-, 2,4-Butylen beispielhaft und vorzugsweise genannt seien.

Die Alkylenketten können ihrerseits einen oder mehrere vorzugsweise 1 bis 3, insbesondere 1 oder 2 gleiche oder verschiedene Substituenten tragen. Als Substituenten seien beispielhaft und vorzugsweise genannt, gegebenenfalls substituiertes Phenyl und Benzyl.

Die geradkettige oder verzweigte Alkylenkette in den Definitionen von A enthalten vorzugsweise 1 bis 6, insbesondere 1 bis 4 und vor allem 1 bis 3 Kohlenstoffatome, wobei Methylen, 1,1- und 1,2-Ethylen, 1,1-, 1,2-, 1,3- und 2,2-Propylen beispielhaft und vorzugsweise genannt seien.

Aryl steht im folgenden, falls nicht anders definiert, vorzugsweise für gegebenenfalls einfach bis fünffach, gleich oder verschieden substituiertes Aryl mit 6 bis 10 Kohlenstoffatomen, insbesondere für gegebenenfalls einfach bis fünffach und vor allem einfach bis dreifach, gleich oder verschieden substituiertes Phenyl und Naphthyl.

Als Arylsubstituenten seien beispielhaft und vorzugsweise genannt:
Halogen, wie Fluor, Chlor, Brom, Iod insbesondere Fluor, Chlor und Brom; Cyano; Nitro; Alkyl, Alkoxy und Alkylthio mit vorzugsweise 1 bis 4 Kohlenstoffatomen und vor allem Methyl, Ethyl, n- und i-Propyl, n-, i-, s- und t-Butyl, Methoxy, Ethoxy, n- und i-Propoxy und Methylthio; Halogenalkyl, Halogenalkoxy und Halogenalkylthio mit vorzugsweise 1 bis 4 Kohlenstoffatomen und 1 bis 9 gleichen oder verschiedenen Halogenatomen, insbesondere Trifluormethyl, Trifluormethoxy und Trifluormethylthio; Amino; Alkylamino und Dialkylamino mit 1 bis 4 Kohlenstoffatomen, insbesondere Dimethylamino.

Bevorzugt sind Verbindungen der Formel (I) in denen

| | |
|---|---|
| $R^1$, $R^2$, $R^3$, $R^4$ und $R^5$ | gleich oder verschieden sind und für Wasserstoff oder geradkettiges oder verzweigtes Alkyl mit 1 bis 6 Kohlenstoffatomen stehen und |
| Q | für eine unsubstituierte oder durch Phenyl und/oder Benzyl substituierte, geradkettige oder verzweigte Alkylenkette mit 1 bis 6 Kohlenstoffatomen steht, wobei die Phenyl- und/oder Benzylreste ihrerseits unsubstituiert oder einfach bis drei- |

fach, gleich oder verschieden durch Halogen; Cyano; Nitro; Alkyl, Alkoxy mit jeweils 1 bis 4 Kohlenstoffatomen und Halogenalkyl und Halogenalkoxy mit 1 bis 4 Kohlenstoffatomen und 1 bis 9 gleich oder verschiedenen Halogenatomen substituiert sind, oder zusammen mit dem Rest $R^4$ und den Stickstoffatomen einen Pyrrolidinring bildet und

A für eine geradkettige oder verzweigte Alkylenkette mit 1 bis 6 Kohlenstoffatomen steht und

n für eine Zahl 0 oder 1 steht und

Ar für unsubstituiertes oder einfach bis fünffach, gleich oder verschieden substituiertes Aryl mit 6 bis 10 Kohlenstoffatmen steht, wobei als Arylsubstituenten infrage kommen: Halogen; Cyano; Nitro; Alkyl, Alkoxy und Alkylthio mit 1 bis 4 Kohlenstoffatomen; Halogenalkyl, Halogenalkoxy und Halogenalkylthio mit 1 bis 4 Kohlenstoffatomen und 1 bis 4 gleichen oder verschiedenen Halogenatomen; Amino; Alkylamino und Dialkylamino mit 1 bis 4 Kohlenstoffatomen.

Besonders bevorzugt sind Verbindungen der Formel (I) in denen

$R^1$, $R^2$, $R^3$, $R^4$ und $R^5$ gleich oder verschieden sind und für Wasserstoff oder geradkettiges oder verzweigtes Alkyl mit 1 bis 4 Kohlenstoffatomen stehen und

Q für eine unsubstituierte oder durch Phenyl und/oder Benzyl substituierte, geradkettige oder verzweigte Alkylenkette mit 1 bis 4 Kohlenstoffatomen steht, wobei die Phenyl- und/oder Benzylreste ihrerseits unsubstituiert oder einfach bis dreifach, gleich oder verschieden durch Fluor, Chlor, Brom, Cyano, Nitro, Methyl, Ethyl, n- und i-Propyl, n-, i-, s- und t-Butyl, Methoxy, Ethoxy, n- und i-Propoxy, Trifluormethyl und Trifluormethoxy substituiert sind;
oder zusammen mit dem Rest $R^4$ und dem Stickstoffatom einen Pyrrolidinring bildet und

A für eine geradkettige oder verzweigte Alkylenkette mit 1 bis 4 Kohlenstoffatomen steht und

n für eine Zahl 0 oder 1, insbesondere 0 steht und

Ar für unsubstituiertes oder einfach bis dreifach, gleich oder verschieden substituiertes Phenyl oder Naphthyl steht wobei als Substituenten infrage kommen: Fluor, Chlor, Brom, Cyano, Nitro, Methyl, Ethyl, n- und i-Propyl, n-, i-, s- und t-Butyl, Methoxy, Ethoxy, n- und i-Propoxy, Trifluormethyl und Trifluormethoxy.

Verwendet man beispielsweise 2,2-Dichlor-1-methyl-cyclopropancarbonsäurechlorid, Glycin und 4-Methylbenzylamin als Ausgangsprodukte, so kann der Ablauf des erfindungsgemäßen Verfahrens a) durch das folgende Formelschema wiedergegeben werden:

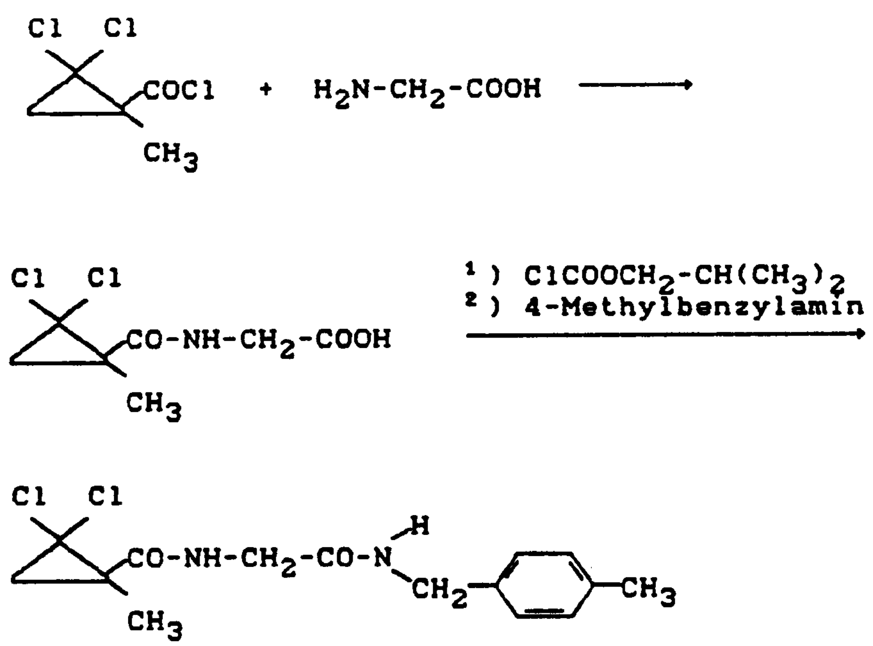

Verwendet man beispielsweise t-Butyloxycarbonyl-Glycin (Boc-Glycin), Benzylamin und 2,2-Dichlor-1-methyl-cyclopropancarbonsäurechlorid als Ausgangsprodukte, so kann der Ablauf des erfindungsgemäßen Verfahrens b) durch das folgende Formelschema wiedergegeben werden:

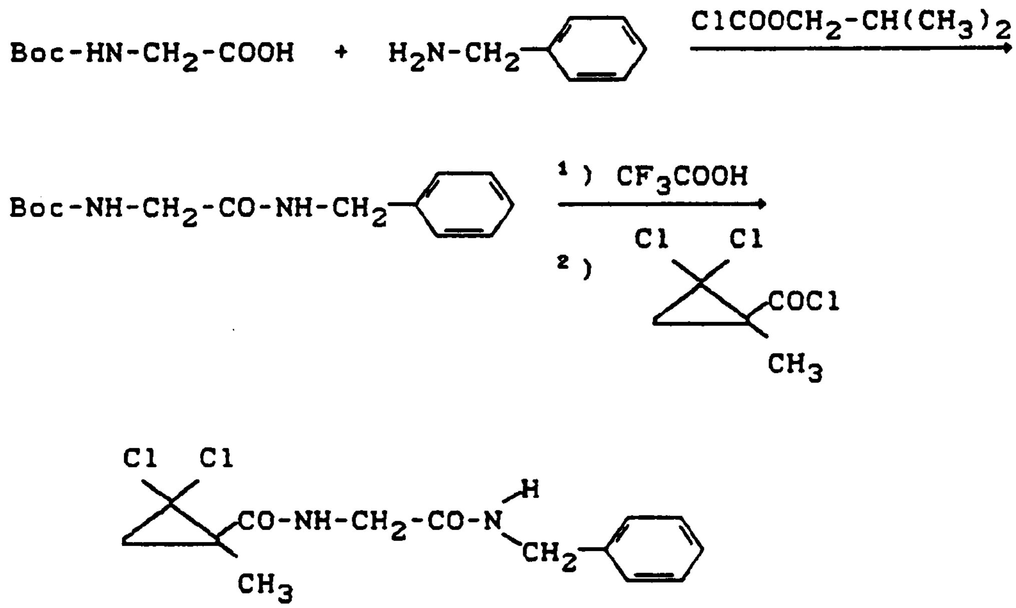


Die für die Duchführung der erfindungsgemäßen Verfahren a) und b) zu verwendenden Cyclopropancarbonsäure-Derivate sind durch die Formel (II) allgemein definiert. In dieser Formel haben $R^1$, $R^2$ und $R^3$ vorzugsweise die Bedeutung, die bereits im Zusammenhang mit der Beschreibung der erfindungsgemäßen Stoffe der Formel (I) für diese Substituenten bevorzugt genannt wurden.

Die Cyclopropancarbonsäure-Derivate sind bekannt (vgl. z.B. EP-A-170 842 und DE-OS-2 219 710) oder können nach den dort angegebenen Verfahren erhalten werden.

Die für die Durchführung des erfindungsgemäßen Verfahrens (a) als Ausgangsstoffe zu verwendenden Aminosäure-Derivate sind durch die Formel (III) allgemein definiert. In dieser Formel haben $R^1$, $R^2$, $R^3$ und X vorzugsweise die Bedeutungen, die bereits im Zusammenhang mit der Beschreibung der erfindungsmäßen Stoffe der Formel (I) für diese Substituenten bevorzugt genannt wurden.

Die Aminosäure-Derivate der Formel (III) sind teilweise bekannt (vgl. z.B. Houben-Weyl, Methoden der organischen Chemie, Band XV, Teil 1 und 2, Georg Thieme Verlag, Stuttgart 1974; bzw. R.C. Sheppard, A Specialist Periodical Report, Amino-acids, Peptids and Proteins, The Royal Society of Chemistry, Burlington House, London 1978, bzw. I.P. Greenstein and M. Winitz, Chemistry of Amino Acids, I. Wiley Sons Inc., New York, London 1961; bzw. E. Schröder and K. Lübke, The Peptides Vol. I, Academic Press, New York, London 1965) oder können nach den dort angegebenen Verfahren erhalten werden.

Die durch eine Aminoschutzgruppe W geschützten Aminosäure-Derivate der Formel (VI) sind ebenfalls bekannt oder können nach bekannten Verfahren hergestellt werden.

Der Ausdruck "Aminoschutzgruppe" ist allgemein bekannt (vgl. z.B.: Houben-Weyl, Methoden der organischen Chemie, Band XV, Teil 1, Georg Thieme Verlag, Stuttgart 1974) und bezieht sich auf Gruppen, die geeignet sind, eine Aminogruppe vor chemischen Umsetzungen zu schützen (zu blockieren), die aber leicht entfernbar sind, nachdem die gewünschte Reaktion an anderen Stellen des Moleküls durchgeführt worden ist. Bevorzugte Aminoschutzgruppe ist tert-Butoxycarbonyl (BOC).

Die Aminoschutzgruppe kann in an sich bekannter Weise nach üblichen Methoden z.B. durch Solvolyse, wie Hydrolyse, Acidolyse, durch Reduktion, wie z.B. durch Hydrogenolyse in Gegenwart eines Hydrierungskatalysators oder mittels eines Reduktionssystems aus Metall und protonenabspaltendem Mittel, wobei je nach Art der Schutzgruppe verschiedenartige (auch andersartige) sowie selektive Abspaltungsmethoden angewendet werden können, gegebenenfalls in Gegenwart eines geeigneten Lösungs- oder Verdünnungsmittels oder eines Gemisches aus ihnen, wobei man je nach Bedarf unter Kühlen, bei Raumtemperatur oder unter Erwärmen, z.B. in einem Temperaturbereich von etwa -10°C bis zur Siedetemperatur des Reaktionsmediums, vorzugsweise von etwa -10°C bis etwa 150°C, und, falls erforderlich, in einem geschlossenen Gefäss, unter

Druck, in einer Inertgasatmosphäre und/oder unter wasserfreien Bedingungen abgespalten werden (vgl. Protective Groups in Organic Synthesis, Th. W. Greene, Wiley Interscience, 1981).

Die Acidolyse gelingt z.B. mit starken Säuren, zweckmäßig mit Trifluoressigsäure oder Perchlorsäure, aber auch mit anderen starken anorganischen Säuren wie Salzsäure oder Schwefelsäure, starken organischen Carbonsäuren wie Trichloressigsäure oder Sulfonsäuren wie Benzol- oder p-Toluolsulfonsäure. Die Anwesenheit eines zusätzlichen inerten Lösungsmittels ist möglich. Als inerte Lösungsmittel eignen sich vorzugsweise organische, beispielsweise Carbonsäuren wie Essigsäure, Ether wie Tetrahydrofuran oder Dioxan, Amide wie Dimethylformamid (DMF), halogenierte Kohlenwasserstoffe wie Dichlormethan, ferner auch Alkohole wie Methanol, Ethanol oder Isopropanol sowie Wasser.

Ferner kommen Gemische der vorgenannten Lösungsmittel in Frage. Trifluoressigsäure wird vorzugsweise im Überschuß ohne Zusatz eines weiteren Lösungsmittels verwendet, Perchlorsäure vorzugsweise in Form eines Gemisches aus Essigsäure und 70 %iger Perchlorsäure im Verhältnis 9 : 1. Die Reaktionstemperaturen für diese Solvolysen liegen zweckmäßig zwischen etwa 0 und etwa 50°C, vorzugsweise arbeitet man zwischen 15 und 30°C (Raumtemperatur).

Die BOC-Gruppe kann z.B. bevorzugt mit 40 %iger Trifluoressigsäure in Methylenchlorid oder mit etwa 3 bis 5 n Salzsäure in Dioxan bei 15 - 30°C abgespalten werden, die FMOC-Gruppe (9-Fluorenylmethyloxycarbonyl) mit einer etwa 5-bis 20%igen Lösung von Dimethylamin, Diethylamin oder Piperidin in Dimethylformamid bei 15 - 30°C.

Als carboxy-aktivierte Derivate der Carbonsäuren der Formeln (II), (IV) und (VI) kommen alle Carboxy-aktivierten Derivate infrage, wie Säurehalogenide, wie z.B. Säurechloride, Säureazide, ferner symmetrische und gemischte Anhydride, wie beispielsweise die gemischten O-Alkylkohlensäureanhydride, weiterhin aktivierte Ester, wie z.B. p-Nitrophenylester oder N-Hydroxisuccinimidester sowie mit Kondensationsmitteln, wie z.B. Dicyclohexylcarbodiimid oder Carbonyldiimidazol, in situ erzeugte aktivierte Formen der Carbonsäuren.

Vorzugsweise werden die den Carbonsäuren der Formeln (II), (IV) und (VI) entsprechenden Säurechloride und gemischten Anhydride eingesetzt. Sie können hergestellt werden, indem man die Carbonsäuren der Formeln (II), (IV) und (VI) oder deren Salze mit einem Halogenierungsmittel oder einem der allgemein bekannten Mittel zur Herstellung von gemischten Anhydriden, wie beispielsweise Phosphorpentachlorid, Thionylchlorid, Oxalylchlorid oder Chlorameisensäureisobutylester, in allgemein bekannter Art und Weise umsetzt (vgl. z.B. Houben-Weyl, Methoden der organischen Chemie, Band XV, Teil 2, Georg Thieme Verlag, Stuttgart 1974; M. Bodanszky, Principles of Peptide Synthesis, Springer-Verlag, Berlin 1984; M. Bodanszky, The Practice of Peptide Synthesis, Springer Verlag, Berlin 1984).

Bevorzugt werden die Carbonsäuren der Formeln (IV) und (VI) mit Chlorameisensäurealkyl - oder arylestern in die carboxy-aktiven, gemischten Anhydride überführt.

Die Umsetzung kann in Gegenwart indifferenter Verdünnungsmittel wie z.B. aromatische, nichtaromatische oder halogenierte Kohlenwasserstoffe wie z.B. Methylenchlorid und Toluol; Ketone, wie z.B. Aceton; Ester, wie z.B. Ethylacetat; Ether, wie z.B. Tetrahydrofuran; Nitrilen, wie z.B. Acetonitril oder deren Mischungen und/oder in Gegenwart eines Säurebindemittels wie vorzugsweise eines tertiären Amins, wie z.B. Triethylamin, Pyridin oder N-Methylpiperidin, bei Temperaturen von -78 bis 100°C, vorzugsweise -60 bis 25°C, durchgeführt werden.

Die außerdem für die Durchführung der erfindungsgemäßen Verfahren a) und b) als Ausgangsstoffe zu verwendenden Amine sind durch die Formel (V) allgemein definiert. In dieser Formel haben $R^5$, A, n und Ar vorzugsweise die Bedeutung, die bereits im Zusammenhang mit der Beschreibung der erfindungsgemäßen Stoffe der Formel (I) für diese Substituenten bevorzugt genannt werden.

Die Amine der Formel (V) sind allgemein bekannte Verbindungen der organischen Chemie.

Als Verdünnungsmittel kommen für das erfindungsgemäße Verfahren (a) inerte, organische Lösungsmittel, wie Ketone, wie Aceton oder Ethylmethylketon; Ester wie Ethyl- oder Methylacetat; Amide wie Dimethylformamid; Nitrile wie Acetonitril; Chlorkohlenwasserstoffe, wie Methylenchlorid oder Tetrachlorkohlenstoff; Kohlenwasserstoffe, wie Toluol oder Ether, wie Tetrahydrofuran sowie gegebenenfalls Wasser und deren Mischungen in Frage.

Als Säurebindemittel kommen für das erfindungsgemäße Verfahren (a) übliche anorganische und organische Säurebinder in Frage. Hierzu gehören vorzugsweise tertiäre Amine, wie Triethylamin, Pyridin oder N-Methylpiperidin sowie anorganische Basen, z.B. Metallhydroxide wie Natriumund Kaliumhydroxid oder Metallcarbonate, wie Natriumcarbonat oder Calciumcarbonat.

Das erfindungsgemäße Verfahren (a) wird gegebenenfalls in Gegenwart eines Katalysators durchgeführt. Beispielsweise genannt seien 4-Dimethylaminopyridin, 1-Hydroxy-benzotriazol oder Dimethylformamid.

Die Temperaturen können bei der Durchführung des Verfahrens (a) in einem größeren Bereich variiert werden. Im allgemeinen arbeitet man zwischen -78 bis +120°C, vorzugsweise bei -60 bis +40°C.

Bei der Durchführung des erfindungsgemäßen Verfahrens (a) arbeitet man vorzugsweise in äquimolaren

Mengen.

Dabei werden die Aminosäurederivate der Formeln (III) und (VI) als reine optische Isomere (D bzw. L-Form) oder als Racemate eingesetzt.

Bei der Durchführung des erfindungsgemäßen Verfahrens (b) arbeitet man vorzugsweise in äquimolaren Mengen.

Die für das erfindungsgemäße Verfahren (b) in Frage kommenden Verdünnungsmittel, Katalysatoren und Säurebindemittel entsprechen denen des Verfahrens (a).

Die als Zwischenprodukt beim erfindungsgemäßen Verfahren a) isolierbaren Cyclopropanoylaminosäure-Derivate der Formel (IV) sind teilweise bekannt (DE-OS 2 219 710). Diese Cyclopropanoylaminosäure-Derivate der allgemeinen Formel (IV)

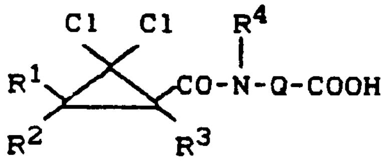

(IV)

in welcher

| | |
|---|---|
| $R^1$, $R^2$, $R^3$, und $R^4$ | gleich oder verschieden sind und für Wasserstoff oder Alkyl stehen und |
| Q | für eine unsubstituierte oder substituierte, geradkettige oder verzweigte Alkylenkette steht, oder zusammen mit dem Rest $R^4$ und dem Stickstoffatom einen Pyrrolidinring bildet, |

ausgenommen die Verbindung, in der $R^1$ und $R^2$ für Methyl und $R^3$ und $R^4$ für Wasserstoff und Q für $-CH_2-$ steht, sind ebenfalls Gegenstand dieser Anmeldung.

Die Verbindungen der Formel (IV) können ebenfalls zur Bekämpfung von Schädlingen, insbesondere als Fungizide im Pflanzenschutz, verwendet werden.

In dieser Formel (IV) haben $R^1$, $R^2$, $R^3$, $R^4$ und Q vorzugsweise die Bedeutung, die bereits im Zusammenhang mit der Beschreibung der erfindungsgemäßen Stoffe der Formel (I) für diese Substituenten bevorzugt genannt wurden.

Die erfindungsgemäßen Wirkstoffe weisen eine starke Wirkung gegen Schädlinge auf und können zur Bekämpfung von unerwünschten Schadorganismen praktisch eingesetzt werden. Die Wirkstoffe sind für den Gebrauch als Pflanzenschutzmittel insbesondere als Fungizide geeignet.

Fungizide Mittel im Pflanzenschutz werden eingesetzt zur Bekämpfung von Plasmodiophoromycetes, Oomycetes, Chytridiomycetes, Zygomycetes, Ascomycetes, Basidiomycetes, Deuteromycetes.

Beispielhaft aber nicht begrenzend seien einige Erreger von pilzlichen Erkrankungen, die unter die oben aufgezählten Oberbegriffe fallen, genannt:

Pythium-Arten, wie beispielsweise Pythium ultimum;
Phytophthora-Arten, wie beispielsweise Phytophthora infestans;
Pseudoperonospora-Arten, wie beispielsweise Pseudoperonospora humuli oder Pseudoperonospora cubensis;
Plasmopara-Arten, wie beispielsweise Plasmopara viticola;
Peronospora-Arten, wie beispielsweise Peronospora pisi oder P. brassicae,
Erysiphe-Arten, wie beispielsweise Erysiphe graminis;
Sphaerotheca-Arten, wie beispielsweise Sphaerotheca fuliginea;
Podosphaera-Arten, wie beispielsweise Podosphaera leucotricha;
Venturia-Arten, wie beispielsweise Venturia inaequalis;
Pyrenophora-Arten, wie beispielsweise Pyrenophora teres oder P. graminea
(Konidienform: Drechslera, Syn: Helminthosporium);
Cochliobolus-Arten, wie beispielsweise Cochliobolus sativus
(Konidienform: Drechslera, Syn: Helminthosporium);
Uromyces-Arten, wie beispielsweise Uromyces appendiculatus;
Puccinia-Arten, wie beispielsweise Puccinia recondita;
Tilletia-Arten, wie beispielsweise Tilletia caries;
Ustilago-Arten, wie beispielsweise Ustilago nuda oder Ustilago avenae;
Pellicularia-Arten, wie beispielsweise Pellicularia sasakii;
Pyricularia-Arten, wie beispielsweise Pyricularia oryzae;

Fusarium-Arten, wie beispielsweise Fusarium culmorum;
Botrytis-Arten, wie beispielsweise Botrytis cinerea;
Septoria-Arten, wie beispielsweise Septoria nodorum;
Leptosphaeria-Arten, wie beispielsweise Leptosphaeria nodorum;
Cercospora-Arten, wie beispielsweise Cercospora canescens;
Alternaria-Arten, wie beispielsweise Alternaria brassicae;
Pseudocercosporella-Arten, wie beispielsweise Pseudocercosporella herpotrichoides.

Die gute Pflanzenverträglichkeit der Wirkstoffe in den zur Bekämpfung von Pflanzenkrankheiten notwendigen Konzentrationen erlaubt eine Behandlung von oberirdischen Pflanzenteilen, von Pflanz- und Saatgut, und des Bodens.

Dabei lassen sich die erfindungsgemäßen Wirkstoffe mit besonders gutem Erfolg zur Bekämpfung von Reiskrankheiten, wie beispielsweise gegen den Erreger der Reisfleckenkrankheit (Pyricularia oryzae) oder gegen den Erreger der Reisstengelkrankheit (Pellicularia sasakii) einsetzen.

Die Wirkstoffe können in Abhängigkeit von ihren jeweiligen physikalischen und/oder chemischen Eigenschaften in übliche Formulierungen übergeführt werden, wie Lösungen, Emulsionen, Suspensionen, Pulver, Schäume, Pasten, Granulate, Aerosole, Feinstverkapselungen in polymeren Stoffen und in Hüllmassen für Saatgut, sowie ULV-Kaltund Warmnebel-Formulierungen.

Diese Formulierungen werden in bekannter Weise hergestellt, z.B. durch Vermischen der Wirkstoffe mit Streckmitteln, also flüssigen Lösungsmitteln, unter Druck stehenden verflüssigten Gasen und/oder festen Trägerstoffen, gegebenenfalls unter Verwendung von oberflächenaktiven Mitteln, also Emulgiermitteln und/oder Dispergiermitteln und/oder schaumerzeugenden Mitteln. Im Falle der Benutzung von Wasser als Streckmittel können z.B. auch organische Lösungsmittel als Hilfslösungsmittel verwendet werden. Als flüssige Lösungsmittel kommen im wesentlichen in Frage: Aromaten, wie Xylol, Toluol, oder Alkylnaphthaline, chlorierte Aromaten oder chlorierte aliphatische Kohlenwasserstoffe, wie Chlorbenzole, Chlorethylene oder Methylenchlorid, aliphatische Kohlenwasserstoffe, wie Cyclohexan oder Paraffine, z.B. Erdölfraktionen, Alkohole, wie Butanol oder Glycol sowie deren Ether und Ester, Ketone, wie Aceton, Methylethylketon, Methylisobutylketon oder Cyclohexanon, stark polare Lösungsmittel, wie Dimethylformamid und Dimethylsulfoxid, sowie Wasser; mit verflüssigten gasförmigen Streckmitteln oder Trägerstoffen sind solche Flüssigkeiten gemeint, welche bei normaler Temperatur und unter Normaldruck gasförmig sind, z.B. Aerosol-Treibgase, wie Halogenkohlenwasserstoffe sowie Butan, Propan, Stickstoff und Kohlendioxid; als feste Trägerstoffe kommen in Frage: z.B. natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate; als feste Trägerstoffe für Granulate kommen in Frage: z.B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Sägemehl, Kokosnußschalen, Maiskolben und Tabakstengel; als Emulgier- und/oder schaumerzeugende Mittel kommen in Frage: z.B. nichtionogene und anionische Emulgatoren, wie Polyoxyethylen-Fettsäure-Ester, Polyoxyethylen-Fettalkohol-Ether, z.B. Alkylarylpolyglykol-Ether, Alkylsulfonate, Alkylsulfate, Arylsulfonate sowie Eiweißhydrolysate; als Dispergiermittel kommen in Frage: z.B. Lignin-Sulfitablaugen und Methylcellulose.

Es können in den Formulierungen Haftmittel wie Carboxymethylcellulose, natürliche und synthetische pulverige, körnige oder latexförmige Polymere verwendet werden, wie Gummiarabicum, Polyvinylalkohol, Polyvinylacetat, sowie natürliche Phospholipide, wie Kephaline und Lecithine, und synthetische Phospholipide. Weitere Additive können mineralische und vegetabile Öle sein.

Es können Farbstoffe wie anorganische Pigmente, z.B. Eisenoxid, Titanoxid, Ferrocyanblau und organische Farbstoffe, wie Alizarin-, Azo- und Metallphthalocyaninfarbstoffe und Spurennährstoffe wie Salze von Eisen, Mangan, Bor, Kupfer, Kobalt, Molybdän und Zink verwendet werden.

Die Formulierungen enthalten im allgemeinen zwischen 0,1 und 95 Gewichtsprozent Wirkstoff, vorzugsweise zwischen 0,5 und 90 %.

Die erfindungsgemäßen Wirkstoffe können in den Formulierungen in Mischung mit anderen bekannten Wirkstoffen vorliegen wie Fungizide, Insektizide, Akarizide und Herbizide sowie in Mischungen mit Düngemitteln und Wachstumsregulatoren.

Die Wirkstoffe können als solche, in Form ihrer Formulierungen oder den daraus bereiteten Anwendungsformen wie gebrauchsfertige Lösungen, Suspensionen, Spritzpulver, Pasten, lösliche Pulver, Stäubemittel und Granulate angewendet werden. Die Anwendung geschieht in üblicher Weise, z.B. durch Gießen, Verspritzen, Versprühen, Verstreuen, Verstäuben, Verschäumen, Bestreichen usw.. Es ist ferner möglich, die Wirkstoffe nach dem Ultra-Low-Volume-Verfahren auszubringen oder die Wirkstoffzübereitung oder den Wirkstoff selbst in den Boden zu injizieren. Es kann auch das Saatgut der Pflanzen behandelt werden.

Bei der Behandlung von Pflanzenteilen können die Wirkstoffkonzentrationen in den Anwendungsformen

in einem größeren Bereich variiert werden. Sie liegen im allgemeinen zwischen 1 und 0,0001 Gew.-%, vorzugsweise zwischen 0,5 und 0,001 %.

Bei der Saatgutbehandlung werden im allgemeinen Wirkstoffmengen von 0,001 bis 50 g je Kilogramm Saatgut, vorzugsweise 0,01 bis 10 g benötigt.

Bei Behandlung des Bodens sind Wirkstoffkonzentrationen von 0,00001 bis 0,1 Gew.-%, vorzugsweise von 0,0001 bis 0,02 % am Wirkungsort erforderlich.

Herstellungsbeispiele:

Beispiel 1

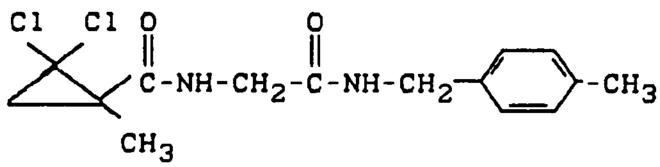

Verfahren a)

5 g N-(2,2-Dichlor-1-methylcyclopropanoyl)-glycin (0,022 Mol), gelöst in 55 ml $CH_2Cl_2$/THF 9:1, werden bei -20°C mit 4,2 g (0,022 Mol) N-Methylpiperidin versetzt. Anschließend tropft man bei -20°C schnell 3,0 g (0,022 Mol) Chlorameisensäureisobutylester zu, rührt 10 Minuten bei gleicher Temperatur nach, kühlt auf -60°C ab und läßt 2,7 g (0,022 Mol) 4-Methylbenzylamin zulaufen, wobei die Temperatur unter -15°C gehalten wird. Nach 2 h bei -15°C läßt man 15 Stunden bei Raumtemperatur nachrühren, filtriert vom Feststoff ab, rührt mit Methylenchlorid nach, engt ein, verrührt den Rückstand mit Wasser, saugt den farblosen Feststoff ab, wäscht erst mit $NaHCO_3$-Lösung, dann mit Wasser nach und trocknet das Produkt im Vakuum bei 50°C.

Man erhält 6,2 g (85 % der Theorie) N-(2,2-Dichlor-1-methylcyclopropanoyl)-glycin-4-methylbenzylamid mit dem Schmelzpunkt von 115-116°C.

Herstellung der Ausgangsprodukte

I.

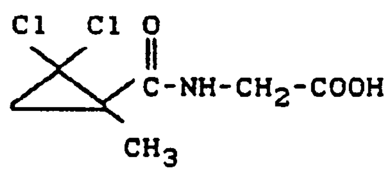

2,0 g (0,027 Mol) Glycin werden in 7 ml 4-N-NaOH gelöst und unter Eiskühlung bei 5 bis 10°C portionsweise mit 2,2-Dichlor-1-methylcyclopropancarbonsäurechlorid und 6,5 ml 4-N-NaOH-Lösung versetzt, wobei der pH-Wert > 8 gehalten wird. Man läßt 30 Minuten nachrühren, extrahiert die Wasserphase 2 mal mit Ether, säuert mit 1-N-HCl auf pH ca. 2 an und saugt den entstandenen Niederschlag ab. Nach dem Trocknen erhält man 4,5 g (74 % der Theorie) N-(2,2-Dichlor-1-methylcyclopropanoyl)-glycin mit einem Schmelzpunkt von 139-140°C.

In analoger Weise werden die folgenden Verbindungen der allgemeinen Formel (IV)

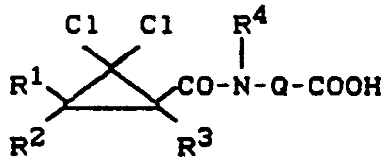

(IV)

erhalten:

**Tabelle 1**

| Nr. | $R^1$ | $R^2$ | $R^3$ | $R^4$ | Q | phys. Daten |
|---|---|---|---|---|---|---|
| II | H | H | $CH_3$ | H | $-CH_2-$ | mp 139-140° C |
| III | H | H | $CH_3$ | $CH_3$ | $-CH_2-$ | mp 68-70° C |
| IV | H | H | $CH_3$ | H | 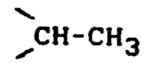 | NMR $\delta$ = 4,2-4,3 ppm |
| V | H | H | $CH_3$ | H | 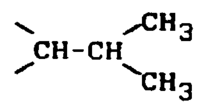 | mp 166-168° C |
| VI | H | H | $CH_3$ | H | 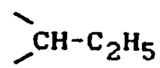 | NMR $\delta$ = 4,05-4,15 ppm |
| VII | H | H | $CH_3$ | H | $-CH_2-CH_2-$ | NMR $\delta$ = 3,0-3,2 ppm |
| VIII | H | H | $CH_3$ | H | 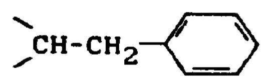 | mp 150-152° C |
| IX | H | H | $CH_3$ | H | 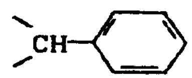 | mp 156-158° C |
| X | H | H | $CH_3$ | H | 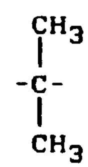 | mp 215-217° C |
| XI | H | H | $CH_3$ | H | $-CH_2-CH_2-CH_2-$ | mp 120-122° C |

Beispiel 2

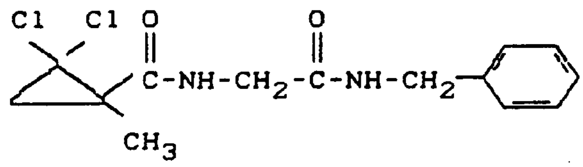

Verfahren b)

Zu 2 g Glycinbenzylamid-hydrotrifluoracetat (0,007 Mol), suspendiert in 60 ml THF, werden bei 0 bis 10°C 2,1 g (0,018 Mol) Triethylamin zugegeben und anschließend im gleichen Temperaturbereich 1,4 g (0,007 Mol) 2,2,-Dichlor-1-methylcyclopropancarbonsäurechlorid, verdünnt mit 15 ml THF, zugetropft. Man läßt 16 Stunden bei Raumtemperatur rühren, engt unter Vakuum ein, versetzt mit 200 ml Wasser, saugt den entstandenen Niederschlag ab, wäscht ihn mit verd. HCl, $NaHCO_3$-Lösung und Wasser und trocknet bei 50°C im Vakuum.

Man erhält 2,0 g (88 % der Theorie) N-(2,2-Dichlor-1-methyl-cyclopropanoyl)-glycinbenzylamid mit dem Schmelzpunkt von 139-141°C.

Herstellung der Ausgangsprodukte

α)

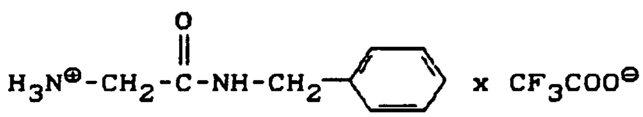

12 g (0,048 Mol) Boc-Glycinbenzylamid werden in 400 ml Trifluoressigsäure 1 Stunde bei Raumtemperatur gerührt und nach Einengen mit Ether verrührt. Der farblose Feststoff wird abgesaugt und mit Ether nachgewaschen und im Vakuum-Exsikkator über NaOH-Plätzchen getrocknet.

Man erhält 10,3 g (77 % der Theorie) Glycinbenzylamidhydrotrifluoracetat (NMR, DMSO δ = 4,35 ppm/7,2-7,4 ppm).

β)

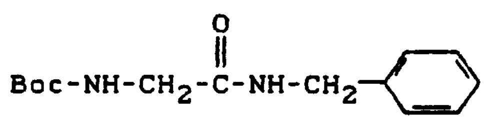

Zu 10 g Boc-Glycin (0,057 Mol), gelöst in 100 ml $CH_2Cl_2$, gibt man bei -20°C 5,7 g (0,057 Mol) N-Methylpiperidin und tropft bei gleicher Temperatur anschließend schnell 7,8 g Chlorameisensäureisobutylester (0,057 Mol) zu. Nach 10 Minuten bei -20°C kühlt man auf -60°C ab, läßt 6,1 g (0,057 Mol) Benzylamin so zulaufen, daß die Temperatur unter -15°C gehalten wird, rührt 2 Stunden bei -15°C nach und läßt auf Raumtemperatur erwärmen. Es wird vom Feststoff abfiltriert, mit $CH_2Cl_2$ nachgewaschen, eingeengt und mit Wasser versetzt. Das entstandene Öl wird mit Essigester extrahiert, die Essigesterphase mit $NaHCO_3$-Lösung und Wasser gewaschen, getrocknet und eingeengt.

Man erhält 12 g (84 % der Theorie) Boc-Glycinbenzylamid (NMR, $CDCl_3$, δ = 3,7 ppm/4,4 ppm/1,4 ppm).

In analoger Weise und entsprechend der erfindungsgemäßen Verfahren werden die nachfolgenden Verbindungen der allgemeinen Formel (I)

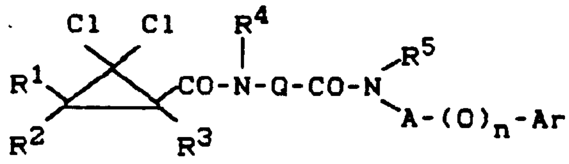

(I)

erhalten.

Tabelle 2

| Beispiel Nr. | $R^1$ | $R^2$ | $R^3$ | $R^5$ | $R^4$ | Q | A | n | Ar | phys. Daten |
|---|---|---|---|---|---|---|---|---|---|---|
| 3 | $CH_3$ | $CH_3$ | H | H | H | $-CH_2-$ | $CH-CH_3$ | o | 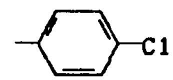 | |
| 4 | H | H | $CH_3$ | H | H | $-CH_2CH_2CH_2-$ | $-CH_2-$ | o | 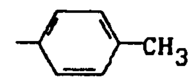 | mp 128-130° C |
| 5 | H | H | $CH_3$ | H | H | $-CH_2-$ | $-CH_2-$ | o | 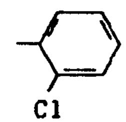 | mp 119-126° C |
| 6 | H | H | $CH_3$ | H | H | $-CH_2-$ | $-CH_2-$ | o | 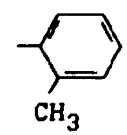 | mp 109-112° C |
| 7 | H | H | $CH_3$ | H | H | $-CH_2-$ | $-CH_2-$ | o | 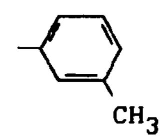 | mp 92- 93° C |

<u>Tabelle 2</u> - Fortsetzung -

| Beispiel Nr. | $R^1$ | $R^2$ | $R^3$ | $R^5$ | $R^4$ | Q | A | n | Ar | phys. Daten |
|---|---|---|---|---|---|---|---|---|---|---|
| 8 | H | H | $CH_3$ | H | H | $-CH_2-$ | $CH-CH_3$ | o | 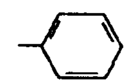 | mp 108-113°C |
| 9 | H | H | $CH_3$ | H | H | $-CH_2-$ | $-CH_2CH_2-$ | o | 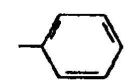 | mp 95- 97°C |
| 10 | H | H | $CH_3$ | H | H | $-CH_2-$ | $-CH_2-$ | o | 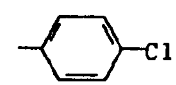 | mp 115-116°C |
| 11 | H | H | $CH_3$ | H | H | $-CH_2-$ | $-CH_2-$ | o | 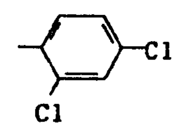 | NMR/DMSO δ: 1,5/4,3/ 7,3-7,4 ppm |
| 12 | H | H | $CH_3$ | H | H | $-CH_2CH_2CH_2-$ | $CH-CH_3$ | o | 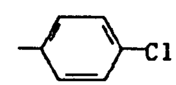 | mp 139-141°C |

**Tabelle 2** - Fortsetzung -

| Beispiel Nr. | $R^1$ | $R^2$ | $R^3$ | $R^5$ | $R^4$ | Q | A | n | Ar | phys. Daten |
|---|---|---|---|---|---|---|---|---|---|---|
| 13 | H | H | $CH_3$ | H | H | 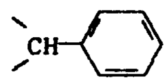 | $CH-CH_3$ | o | 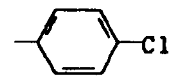 | mp 200-204° C |
| 14 | H | H | $CH_3$ | H | H | 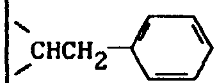 | $CH-CH_3$ | o | 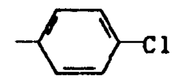 | mp 168-170° C |
| 15 | H | H | $CH_3$ | H | H | $-CH_2CH_2-$ | $CH-CH_3$ | o | 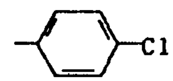 | mp 161-163° C |
| 16 | H | H | $CH_3$ | H | H | $CH-CH(CH_3)_2$ | $CH-CH_3$ | o | 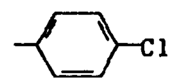 | mp 189-191° C |
| 17 | H | H | $CH_3$ | H | $CH_2$ | $-CH_2-$ | $CH-CH_3$ | o | 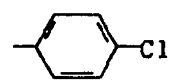 | NMR/DMSO δ: 1,5/ 7,3-7,4 ppm |

<u>Tabelle 2</u> - Fortsetzung -

| Beispiel Nr. | $R^1$ | $R^2$ | $R^3$ | $R^5$ | $R^4$ | Q | A | n | Ar | phys. Daten |
|---|---|---|---|---|---|---|---|---|---|---|
| 18 | H | H | $CH_3$ | H | H | $-CH_2-$ | $-CH_2-$ | o | 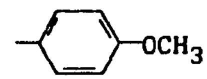 | mp 82- 84° C |
| 19 | H | H | $CH_3$ | H | H | $-CH_2-$ | $-CH_2-$ | o | 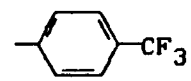 | mp 117-120° C |
| 20 | H | H | $CH_3$ | H | H | $-CH_2-$ | $-CH_2CH_2-$ | o | 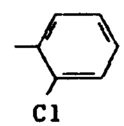 | mp 118-121° C |
| 21 | H | H | $CH_3$ | H | H | $-CH_2-$ | $-CH_2CH_2-$ | o | 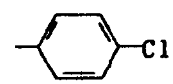 | NMR/DMSO δ: 1,5/ 3,6-3,7/ 7,2-7,4 |
| 22 | H | H | $CH_3$ | H | H | $-CH_2-$ | $-CH_2-$ | o | 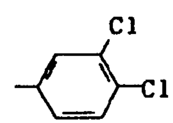 | mp 123-125° C |

<u>Tabelle 2</u> - Fortsetzung -

| Beispiel Nr. | $R^1$ | $R^2$ | $R^3$ | $R^5$ | $R^4$ | Q | A | n | Ar | phys. Daten |
|---|---|---|---|---|---|---|---|---|---|---|
| 23 | H | H | $CH_3$ | H | H | $-CH_2-$ | $-CH_2-$ | o | 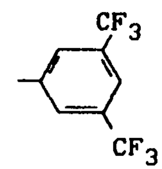 | mp 165-168° C |
| 24 | H | H | $CH_3$ | H | H | $-CH_2-$ | $-CH_2-$ | o | 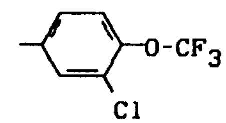 | mp 97- 98° C |
| 25 | H | H | $CH_3$ | H | H | $-CH_2-$ | $-CH_2-$ | o | 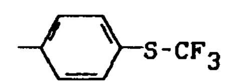 | NMR/DMSO δ: 1,5 / 4,3 / 7,5-7,6 |
| 26 | H | H | $CH_3$ | H | H | $-CH_2-$ | $-CH_2-$ | o | 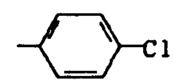 | mp 115-118° C |
| 27 | H | H | $CH_3$ | H | H | $-CH_2-$ | $-CH_2CH_2-$ | 1 | 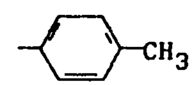 | mp 91- 92° C |

**Tabelle 2** - Fortsetzung -

| Beispiel Nr. | $R^1$ | $R^2$ | $R^3$ | $R^5$ | $R^4$ | Q | A | n | Ar | phys. Daten |
|---|---|---|---|---|---|---|---|---|---|---|
| 28 | H | H | $CH_3$ | $CH_3$ | H | $-CH_2-$ | $>CH-CH_3$ | o | 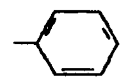 | NMR/DMSO δ: 1,6 / 4,1 / 7,2 - 7,4 |
| 29 | H | H | $CH_3$ | H | H | $-CH_2-$ | 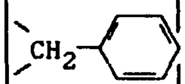 | o | 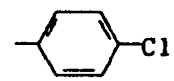 | mp 140-142°C |
| 30 | H | H | $CH_3$ | H | H | 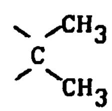 | $>CH-CH_3$ | o | 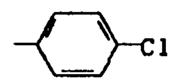 | mp 141-142°C |
| 31 | $CH_3$ | $CH_3$ | $CH_3$ | H | H | $-CH_2-$ | $>CH-CH_3$ | o | 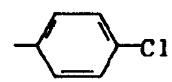 | NMR/DMSO δ: 1,1-1,4 / 3,6-3,8 / 7,3-7,4 |
| 32 | H | H | $CH_3$ | H | H | $-CH_2-$ | $>CHCH_3$ | o | 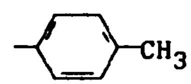 | NMR/DMSO δ: 1,5/3,6-3,7/ 7,0-7,2 |
| 33 | H | H | $CH_3$ | H | H | $-CH_2-$ | $>CH-CH_3$ | o | 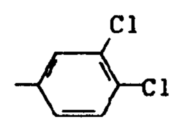 | mp 122-124°C |

<u>Tabelle 2</u> - Fortsetzung -

| Beispiel Nr. | $R^1$ | $R^2$ | $R^3$ | $R^5$ | $R^4$ | Q | A | n | Ar | phys. Daten |
|---|---|---|---|---|---|---|---|---|---|---|
| 34 | H | H | $CH_3$ | H | H | $-CH_2-$ | $CHCH_2CH_3$ | o | 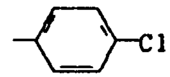 | mp 152-155° C |
| 35 | H | H | $CH_3$ | H | H | $-CH_2CH_2CH_2-$ | $CH-CH_3$ | o | 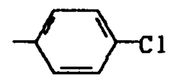 | mp 146-151° C |
| 36 | H | H | $CH_3$ | H | H | $-CH_2-$ | $CH-CH_3$ | o | 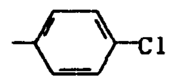 | mp 114-115° C |
| 37 | H | H | $CH_3$ | H | H | 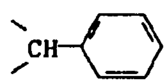 | $CH-CH_3$ | o | 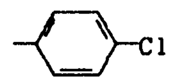 | mp 192-193° C |
| 38 | H | H | $CH_3$ | H | H | 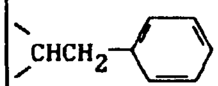 | $CH-CH_3$ | o | 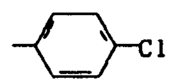 | mp 193-197° C |
| 39 | H | H | $CH_3$ | H | H | $-CH_2CH_2-$ | $CH-CH_3$ | o | 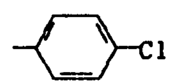 | mp 167-168° C |

**Tabelle 2** - Fortsetzung -

| Beispiel Nr. | $R^1$ | $R^2$ | $R^3$ | $R^5$ | $R^4$ | Q | A | n | Ar | phys. Daten |
|---|---|---|---|---|---|---|---|---|---|---|
| 40 | H | H | $CH_3$ | H | H | 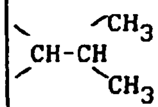 | $CH-CH_3$ | o | 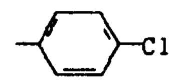 | mp 199-201° C |
| 41 | H | H | $CH_3$ | H | $CH_3$ | $-CH_2-$ | $CH-CH_3$ | o | 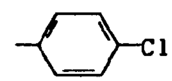 | NMR/DMSO δ: 1,5 / 3,1 / 7,3-7,5 |
| 42 | H | H | $CH_3$ | H | H | $-CH_2-$ | $CH-CH_3$ | o | 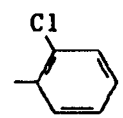 | mp 155-156° C |
| 43 | H | H | $CH_3$ | H | H | $-CH_2-$ | $CH-CH_3$ | o | 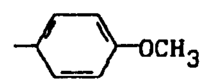 | NMR/DMSO δ: 1,5/3,1-3,3/ 7,4 |
| 44 | H | H | $CH_2CH_2CH_3$ | H | H | $-CH_2-$ | $CH-CH_3$ | o | 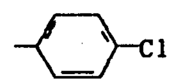 | mp 106-110° C |
| 45 | H | H | $CH_3$ | H | 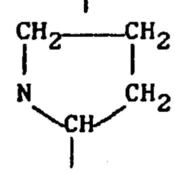 | | $CH-CH_3$ | o | 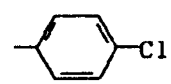 | NMR/DMSO δ: 1,5/3,6-3,7/ 7,3-7,2 |

Tabelle 2 - Fortsetzung -

| Beispiel Nr. | $R^1$ | $R^2$ | $R^3$ | $R^5$ | $R^4$ | Q | A | n | Ar | phys. Daten |
|---|---|---|---|---|---|---|---|---|---|---|
| 46 | H | H | $CH_3$ | H | H | $>CH-CH_3$ | $>CH-CH_3$ | o | 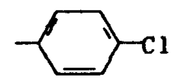 | mp 205-206° C |
| 47 | H | H | $CH_3$ | H | H | $>CHCH_2-$ 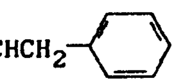 | $>CH-CH_3$ | o | 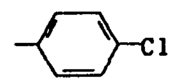 | mp 192-194° C |
| 48 | H | H | $CH_3$ | H | H | $>CH-CH<{CH_3 \atop CH_3}$ | $>CH-CH_3$ | o | 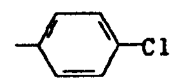 | mp 192-196° C |
| 49 | H | $CH_3$ | $CH_2CH_3$ | H | H | $-CH_2-$ | $>CH-CH_3$ | o | 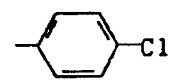 | NMR/DMSO δ: 0,9/3,6-3,8/ 7,3-7,4 |
| 50 | H | H | $CH_3$ | H | H | $>C<{CH_3 \atop CH_3}$ | $>CH-CH_3$ | o | 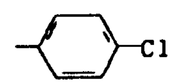 | mp 150-151° C |
| 51 | H | H | $CH<{CH_3 \atop CH_3}$ | H | H | $-CH_2-$ | $>CH-CH_3$ | o | 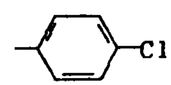 | NMR/DMSO δ: 1,0-1,1/ 3,5-3,9/ 7,3-7,4 |

Tabelle 2 - Fortsetzung -

| Beispiel Nr. | $R^1$ | $R^2$ | $R^3$ | $R^5$ | $R^4$ | Q | A | n | Ar | phys. Daten |
|---|---|---|---|---|---|---|---|---|---|---|
| 52 | H | H | $CH_3$ | H | H | $CH\ CH_2\ CH_3$ | $CH-CH_3$ | o | 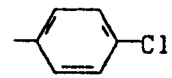 | mp 138-143° C |
| 53 | H | H | $CH_3$ | H | H | $CH\ CH_2\ CH_3$ | $CH-CH_3$ | o | 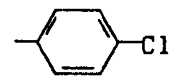 | mp 140-145° C |
| 54 | H | H | $CH_3$ | H | H | $CH-CH_3$ | $CH-CH_3$ | o | 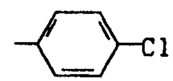 | mp 152-157° C |
| 55 | H | H | $CH_3$ | H | H | $CH-CH_3$ | $CH-CH_3$ | o | 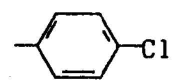 | mp 165-179° C |
| 56 | H | H | $CH_3$ | H | H | $-CH_2-$ | $-CH_2-$ | o | 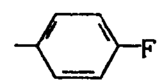 | mp 127-129° C |
| 57 | H | H | $CH_3$ | H | H | $-CH_2-$ | $CH-CH_3$ | o | 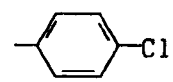 | mp 114-115° C R-(+)-Amin |

Beispiel

Pyricularia-Test (Reis) / systemisch

Lösungsmittel: 12,5 Gewichtsteile Aceton
Emulgator: 0,3 Gewichtsteile Alkylarylpolyglykolether

Zur Herstellung einer zweckmäßigen Wirkstoffzubereitung vermischt man 1 Gewichtsteil Wirkstoff mit den angegebenen Mengen Lösungsmittel und verdünnt das Konzentrat mit Wasser und der angegebenen Menge Emulgator auf die gewünschte Konzentration.

Zur Prüfung auf systemische Eigenschaften werden 40 ml der Wirkstoffzubereitung auf Einheitserde gegossen, in der junge Reispflanzen angezogen werden. 7 Tage nach der Behandlung werden die Pflanzen mit einer wäßrigen Sporensuspension von Pyricularia oryzae inokuliert. Danach verbleiben die Pflanzen in einem Gewächshaus bei einer Temperatur von 25°C und einer rel. Luftfeuchtigkeit von 100 % bis zur Auswertung.

4 Tage nach der Inokulation erfolgt die Auswertung des Krankheitsbefalls.

Die Verbindungen der Herstellungsbeispiele 3, 4, 5, 6, 9, 10, 20, 36, 51 und 57 zeigen bei einer beispielhaften Aufwandmenge von 100 mg Wirkstoff pro 100 $cm^3$ einen ausgezeichneten Wirkungsgrad.

## Patentansprüche

1. Cyclopropanoylaminosäureamid-Derivate der allgemeinen Formel (I)

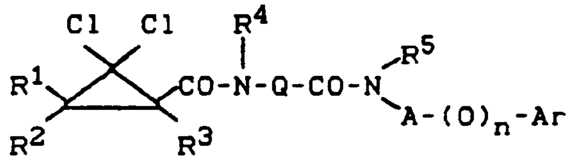

(I)

in welcher

| | |
|---|---|
| $R^1$, $R^2$, $R^3$, $R^4$ und $R^5$ | gleich oder verschieden sind und für Wasserstoff oder Alkyl stehen; |
| Q | für eine unsubstituierte oder substituierte, geradkettige oder verzweigte Alkylenkette steht, oder zusammen mit dem Rest $R^4$ und dem Stickstoffatom einen Pyrrolidinring bildet; |
| A | für eine geradkettige oder verzweigte Alkylenkette steht; |
| n | für eine Zahl 0 oder 1 steht und |
| Ar | für unsubstituiertes oder substituiertes Aryl steht. |

2. Cyclopropanoylaminosäureamid-Derivate der Formel (I) gemäß Anspruch 1, in welcher

| | |
|---|---|
| $R^1$, $R^2$, $R^3$, $R^4$ und $R^5$ | gleich oder verschieden sind und für Wasserstoff oder geradkettiges oder verzweigtes Alkyl mit 1 bis 6 Kohlenstoffatomen stehen und |
| Q | für eine unsubstituierte oder durch Phenyl und/oder Benzyl substituierte, geradkettige oder verzweigte Alkylenkette mit 1 bis 6 Kohlenstoffatomen steht, wobei die Phenyl- und/oder Benzylreste ihrerseits unsubstituiert oder einfach bis dreifach, gleich oder verschieden durch Halogen; Cyano; Nitro; Alkyl, Alkoxy mit jeweils 1 bis 4 Kohlenstoffatomen und Halogenalkyl und Halogenalkoxy mit 1 bis 4 Kohlenstoffatomen und 1 bis 9 gleich oder verschiedenen Halogenatomen substituiert sind, oder zusammen mit dem Rest $R^4$ und dem Stickstoffatom einen Pyrrolidinring bildet und |
| A | für eine geradkettige oder verzweigte Alkylenkette mit 1 bis 6 Kohlenstoffatomen steht und |
| n | für eine Zahl 0 oder 1 steht und |
| Ar | für unsubstituiertes oder einfach bis fünffach, gleich oder verschieden substituiertes Aryl mit 6 bis 10 Kohlenstoffatmen steht, wobei als Arylsubstituenten infrage kommen: Halogen; Cyano; Nitro; Alkyl, Alkoxy und Alkylthio mit 1 bis 4 Kohlenstoffatomen; Halogenalkyl, Halogenalkoxy und |

Halogenalkylthio mit 1 bis 4 Kohlenstoffatomen und 1 bis 4 gleichen oder verschiedenen Halogenatomen; Amino; Alkylamino und Dialkylamino mit 1 bis 4 Kohlenstoffatomen.

**3.** Cyclopropanoylaminosäureamid-Derivate der Formel (I) und gemäß Anspruch 1, in welcher

| | |
|---|---|
| $R^1$, $R^2$, $R^3$, $R^4$ und $R^5$ | gleich oder verschieden sind und für Wasserstoff oder geradkettiges oder verzweigtes Alkyl mit 1 bis 4 Kohlenstoffatomen stehen und |
| Q | für eine unsubstituierte oder durch Phenyl und/oder Benzyl substituierte, geradkettige oder verzweigte Alkylenkette mit 1 bis 4 Kohlenstoffatomen steht, wobei die Phenyl- und/oder Benzylreste ihrerseits unsubstituiert oder einfach bis dreifach, gleich oder verschieden durch Fluor, Chlor, Brom, Cyano, Nitro, Methyl, Ethyl, n- und i-Propyl, n-, i-, s- und t-Butyl, Methoxy, Ethoxy, n- und i-Propoxy, Trifluormethyl und Trifluormethoxy substituiert sind; oder zusammen mit dem Rest $R^4$ und dem Stickstoffatom einen Pyrrolidinring bildet und |
| A | für eine geradkettige oder verzweigte Alkylenkette mit 1 bis 4 Kohlenstoffatomen steht und |
| n | für eine Zahl 0 oder 1 und |
| Ar | für unsubstituiertes oder einfach bis dreifach, gleich oder verschieden substituiertes Phenyl oder Naphthyl steht wobei als Substituenten infrage kommen: Fluor, Chlor, Brom, Cyano, Nitro, Methyl, Ethyl, n- und i-Propyl, n-, i-, s- und t-Butyl, Methoxy, Ethoxy, n-und i-Propoxy, Trifluormethyl und Trifluormethoxy. |

**4.** Verfahren zur Herstellung von Cyclopropanoylaminosäureamid-Derivaten der allgemeinen Formel (I)

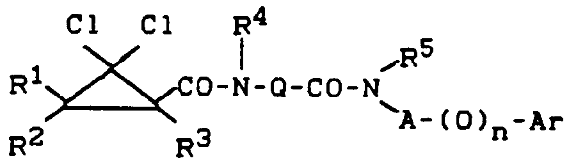

(I)

in welcher

| | |
|---|---|
| $R^1$, $R^2$, $R^3$, $R^4$ und $R^5$ | gleich oder verschieden sind und für Wasserstoff oder Alkyl stehen; |
| Q | für eine unsubstituierte oder substituierte, geradkettige oder verzweigte Alkylenkette steht, oder zusammen mit dem Rest $R^4$ und dem Stickstoffatom einen Pyrrolidinring bildet; |
| A | für eine geradkettige oder verzweigte Alkylenkette steht; |
| n | für eine Zahl 0 oder 1 steht und |
| Ar | für unsubstituiertes oder substituiertes Aryl steht, |

dadurch gekennzeichnet, daß man

a) Cyclopropancarbonsäure der Formel (II)

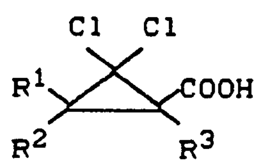

(II)

in welcher

$R^1$, $R^2$, und $R^3$ die oben angegebene Bedeutung haben bzw. deren carboxy-aktivierte Derivate, mit Aminosäure der Formel (III)

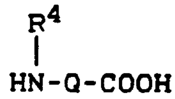 (III)

in welcher
$R^4$ und Q die oben angegebene Bedeutung haben, gegebenenfalls in Gegenwart eines Katalysators, gegebenenfalls in Gegenwart eines Säurebindemittels und gegebenenfalls in Gegenwart eines Verdünnungsmittels umsetzt, und die so erhaltenen Cyclopropanoylaminosäure der Formel (IV)

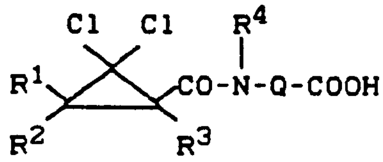 (IV)

in welcher
$R^1$, $R^2$, $R^3$, $R^4$ und Q die oben angegebene Bedeutung haben anschließend, gegebenenfalls nach Überführung in eine carboxy-aktivierte Form, mit einem Amin der Formel (V)

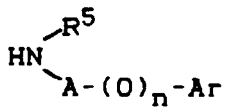 (V)

in welcher
$R^5$, A, n und Ar die oben angegebene Bedeutung haben, gegebenenfalls in Gegenwart eines Katalysators, gegebenenfalls in Gegenwart eines Säurebindemittels und gegebenenfalls in Gegenwart eines Verdünnungsmittels umsetzt,
oder
b) eine durch eine Aminoschutzgruppe geschützte Aminosäure der Formel (VI)

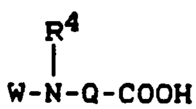 (VI)

in welcher $R^4$ und Q die oben angegebene Bedeutung haben und W für eine Aminoschutzgruppe steht, bzw. deren carboxy-aktivierte Derivate, gegebenenfalls in Gegenwart eines Katalysators, gegebenenfalls in Gegenwart eines Säurebindemittels und gegebenenfalls in Gegenwart eines Verdünnungsmittels mit einem Amin der Formel (V)

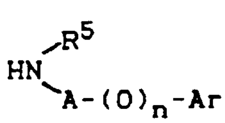 (V)

in welcher
$R^5$, A, n und Ar die oben angegebene Bedeutung haben, umsetzt und anschließend das so erhaltene Aminosäureamid der Formel (VII)

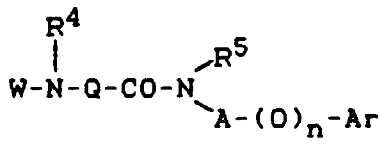

(VII)

in welcher
W, $R^4$, Q, $R^5$, A, n und Ar die oben angegebene Bedeutung haben, nach Abspaltung der Aminoschutzgruppe W mit Cyclopropancarbonsäure der Formel (II)

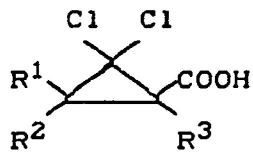

(II)

in welcher
$R^1$, $R^2$ und $R^3$ die oben angegebene Bedeutung haben, bzw. deren carboxy-aktivierten Derivaten, gegebenenfalls in Gegenwart eines Katalysator, gegebenenfalls in Gegenwart eines Säurebindemittels und gegebenenfalls in Gegenwart eines Verdünnungsmitels umsetzt.

**5.** Schädlingsbekämpfungsmittel, gekennzeichnet durch einen Gehalt an mindestens einem Cyclopropanoylaminosäureamid-Derivat der Formel (I) nach den Ansprüchen 1 bis 4.

**6.** Verwendung von Cyclopropanoylaminosäureamid-Derivaten der Formel (I) nach den Ansprüchen 1 bis 4 zur Bekämpfung von Schädlingen.

**7.** Verfahren zur Bekämpfung von Schädlingen, dadurch gekennzeichnet, daß man Cyclopropanoylaminosäureamid-Derivate der Formel (I) nach den Ansprüchen 1 bis 4 auf Schädlingen und/oder ihren Lebensraum einwirken läßt.

**8.** Verfahren zur Herstellung von Schädlingsbekämpfungsmitteln, dadurch gekennzeichnet, daß man Cyclopropanoylaminosäureamid-Derivate der Formel (I) nach den Ansprüchen 1 bis 4 mit Streckmitteln und/oder oberflächenakiven Mitteln vermischt.

**9.** Cyclopropanoylaminosäure-Derivate der allgemeinen Formel (IV)

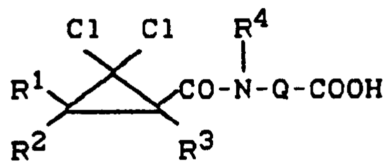

(IV)

in welcher

| | |
|---|---|
| $R^1$, $R^2$, $R^3$, und $R^4$ | gleich oder verschieden sind und für Wasserstoff oder Alkyl stehen und |
| Q | für eine unsubstituierte oder substituierte, geradkettige oder verzweigte Alkylenkette steht, oder zusammen mit dem Rest $R^4$ und dem Stickstoffatom einen Pyrrolidinring bildet, |

ausgenommen die Verbindung, in der $R^1$ und $R^2$ für Methyl und $R^3$ und $R^4$ für Wasserstoff und Q für $-CH_2-$ steht.

## Claims

**1.** Cyclopropanoylamino acid amide derivatives of the general formula (I)

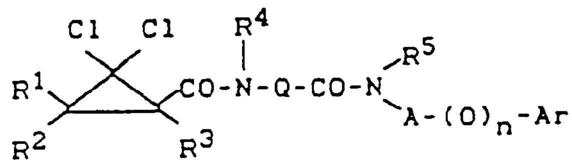

(I)

in which

| | |
|---|---|
| $R^1$, $R^2$, $R^3$, $R^4$ and $R^5$ | are identical or different and represent hydrogen or alkyl; |
| Q | represents an unsubstituted or substituted, straight-chain or branched alkylene chain, or together with the radical $R^4$ and the nitrogen atom forms a pyrrolidine ring; |
| A | represents a straight-chain or branched alkylene chain; |
| n | represents the number 0 or 1 and |
| Ar | represents unsubstituted or substituted aryl. |

2. Cyclopropanoylamino acid amide derivatives of the formula (I) according to Claim 1, in which

| | |
|---|---|
| $R^1$, $R^2$, $R^3$, $R^4$ and $R^5$ | are identical or different and represent hydrogen or straight-chain or branched alkyl having 1 to 6 carbon atoms and |
| Q | represents a straight-chain or branched alkylene chain having 1 to 6 carbon atoms which is unsubstituted or substituted by phenyl and/or benzyl, the phenyl and/or benzyl radicals in turn being unsubstituted or substituted by one to three identical or different substituents from the group comprising halogen; cyano; nitro; alkyl and alkoxy having in each case 1 to 4 carbon atoms and halogenoalkyl and halogenoalkoxy having 1 to 4 carbon atoms and 1 to 9 identical or different halogen atoms, or together with the radical $R^4$ and the nitrogen atom forms a pyrrolidine ring, and |
| A | represents a straight-chain or branched alkylene chain having 1 to 6 carbon atoms and |
| n | represents the number 0 or 1 and |
| Ar | represents aryl having 6 to 10 carbon atoms which is unsubstituted or substituted by one to five identical or different substituents, possible substituents on the aryl being: halogen; cyano; nitro; alkyl, alkoxy and alkylthio having 1 to 4 carbon atoms; halogenoalkyl, halogenoalkoxy and halogenoalkylthio having 1 to 4 carbon atoms and 1 to 4 identical or different halogen atoms; amino; alkylamino and dialkylamino having 1 to 4 carbon atoms. |

3. Cyclopropanoylamino acid amide derivatives of the formula (I) and according to Claim 1, in which

| | |
|---|---|
| $R^1$, $R^2$, $R^3$, $R^4$ and $R^5$ | are identical or different and represent hydrogen or straight-chain or branched alkyl having 1 to 4 carbon atoms and |
| Q | represents a straight-chain or branched alkylene chain having 1 to 4 carbon atoms which is unsubstituted or substituted by phenyl and(or benzyl, the phenyl and(or benzyl radicals in turn being unsubstituted or substituted by one to three identical or different substituents from the group comprising fluorine, chlorine, bromine, cyano, nitro, methyl, ethyl, n- and i-propyl, n-, i-, s- and t-butyl, methoxy, ethoxy, n- and i-propoxy, trifluoromethyl and trifluoromethoxy; or together with the radical $R^4$ and the nitrogen atom forms a pyrrolidine ring, and |
| A | represents a straight-chain or branched alkylene chain having 1 to 4 carbon atoms and |
| n | represents the number 0 or 1, and |
| Ar | represents phenyl or naphthyl which is unsubstituted or substituted by one to three identical or different substituents, possible substituents being: fluorine, chlorine, bromine, cyano, nitro, methyl, ethyl, n- and i-propyl, n-, i-, s- and t-butyl, methoxy, ethoxy, n- and i-propoxy, trifluoromethyl and tri- |

fluoromethoxy.

4. Process for the preparation of cyclopropanoylamino acid amide derivatives of the general formula (I)

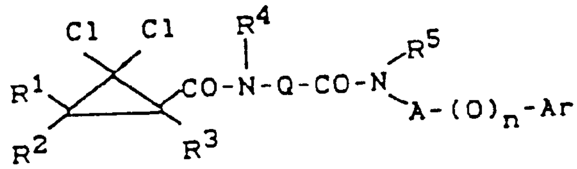

(I)

in which

| | |
|---|---|
| $R^1$, $R^2$, $R^3$, $R^4$ and $R^5$ | are identical or different and represent hydrogen or alkyl; |
| Q | represents an unsubstituted or substituted, straight-chain or branched alkylene chain, or together with the radical $R^4$ and the nitrogen atom forms a pyrrolidine ring; |
| A | represents a straight-chain or branched alkylene chain; |
| n | represents the number 0 or 1 and |
| Ar | represents unsubstituted or substituted aryl, |

characterized in that

a) cyclopropanecarboxylic acid of the formula (II)

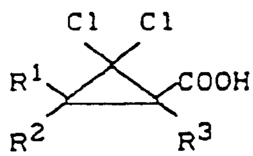

(II)

in which

$R^1$, $R^2$ and $R^3$ have the abovementioned meaning, or carboxy-activated derivatives thereof, is reacted with an amino acid of the formula (III)

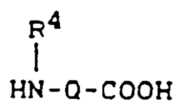

(III)

in which

$R^4$ and Q have the abovementioned meaning, if appropriate in the presence of a catalyst, if appropriate in the presence of an acid-binding agent and if appropriate in the presence of a diluent, and the resulting cyclopropanoylamino acid of the formula (IV)

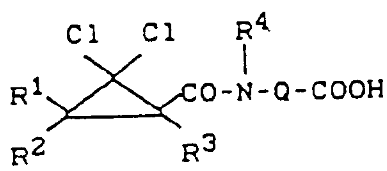

(IV)

in which

$R^1$, $R^2$, $R^3$, $R^4$ and Q have the abovementioned meaning, is then reacted, if appropriate after conversion into a carboxy-activated form, with an amine of the formula (V)

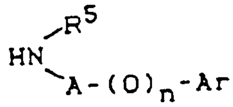

(V)

in which

$R^5$, A, n and Ar have the abovementioned meaning, if appropriate in the presence of a catalyst, if appropriate in the presence of an acid-binding agent and if appropriate in the presence of a diluent,

or

b) an amino acid, protected by an amino-protective group, of the formula (VI)

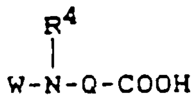

(VI)

in which $R^4$ and Q have the abovementioned meaning and W represents an amino-protective group, or carboxy-activated derivatives thereof, is reacted with an amine of the formula (V)

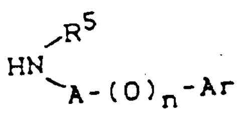

(V)

in which

$R^5$, A, n and Ar have the abovementioned meaning, if appropriate in the presence of a catalyst, if appropriate in the presence of an acid-binding agent and if appropriate in the presence of a diluent, and the resulting amino acid amide of the formula (VII)

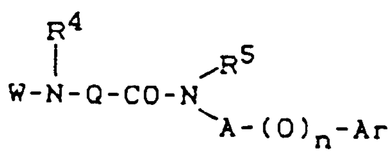

(VII)

in which

W, $R^4$, Q, $R^5$, A, n and Ar have the abovementioned meaning, is then reacted, after the amino-protective group W has been split off, with a cyclopropanecarboxylic acid of the formula (II)

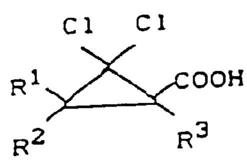

(II)

in which

$R^1$, $R^2$ and $R^3$ have the abovementioned meaning, or carboxy-activated derivatives thereof, if appropriate in the presence of a catalyst, if appropriate in the presence of an acid-binding agent and if appropriate in the presence of a diluent.

**5.** Agents for combating pests, characterized in that they contain at least one cyclopropanoylamino acid amide derivative of the formula (I) according to Claims 1 to 4.

**6.** Use of cyclopropanoylamino acid amide derivatives of the formula (I) according to Claims 1 to 4 for combating pests.

**7.** Method of combating pests, characterized in that cyclopropanoylamino acid amide derivatives of the formula (I) according to Claims 1 to 4 are allowed to act on pests and/or their environment.

**8.** Process for the preparation of agents for combating pests, characterized in that cyclopropanoylamino acid amide derivatives of the formula (I) according to Claims 1 to 4 are mixed with extenders and/or surface-active agents.

**9.** Cyclopropanoylamino acid derivatives of the general formula (IV)

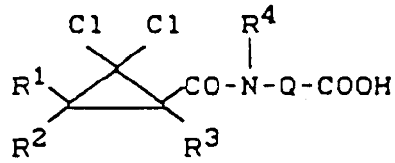

(IV)

in which

$R^1$, $R^2$, $R^3$ and $R^4$ are identical or different and represent hydrogen or alkyl and

Q represents an unsubstituted or substituted, straight-chain or branched alkylene chain, or together with the radical $R^4$ and the nitrogen atom forms a pyrrolidine ring,

excluding the compound in which $R^1$ and $R^2$ represent methyl and $R^3$ and $R^4$ represent hydrogen and Q represents $-CH_2-$.

## Revendications

**1.** Amides dérivés de cyclopropanoylamino-acides de formule générale (I)

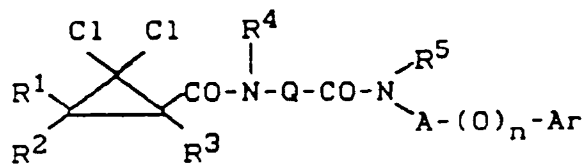

(I)

dans laquelle

$R^1$, $R^2$, $R^3$, $R^4$ et $R^5$ sont identiques ou différents et représentent l'hydrogène ou des restes alkyle ;

Q représente une chaîne alkylénique droite ou ramifiée, non substituée ou substituée, ou forme conjointement avec le reste $R^4$ et l'atome d'azote, un noyau de pyrrolidine ;

A représente une chaîne alkylénique droite ou ramifiée ;

$\underline{n}$ est le nombre 0 ou 1 et

Ar et un groupe aryle non substitué ou substitué.

**2.** Amides dérivés de cyclopropanoylamino-acides de formule (I) suivant la revendication 1, formule dans laquelle

$R^1$, $R^2$, $R^3$, $R^4$ et $R^5$ sont identiques ou différents et représentent l'hydrogène ou un groupe alkyle à chaîne droite ou ramifiée ayant 1 à 6 atomes de carbone,

Q est une chaîne alkylénique droite ou ramifiée ayant 1 à 6 atomes de carbone, non substituée ou substituée par un reste phényle et/ou benzyle, les restes phényle et/ou benzyle étant, quant à eux, non substitués ou portant un à trois substituants, identiques ou différents, halogéno ; cyano ; nitro ; alkyle, alkoxy ayant chacun 1 à 4 atomes de carbone, et halogénalkyle et halogénalkoxy ayant 1 à 4 atomes de carbone et portant 1 à 9 atomes d'halogènes identiques ou différents, ou forme conjointement avec le reste $R^4$ et l'atome d'azote un noyau de pyrrolidine,

A représente une chaîne alkylénique droite ou ramifiée ayant 1 à 6 atomes de carbone,

$\underline{n}$ est le nombre 0 ou 1 et

Ar est un groupe aryle de 6 à 10 atomes de carbone non substitué ou portant

un à cinq substituants identiques ou différents, en considérant comme substituants du reste aryle : un halogène ; un groupe cyano ; nitro ; alkyle ; alkoxy et alkylthio ayant 1 à 4 atomes de carbone ; halogénalkyle, halogénalkoxy et halogénalkylthio ayant 1 à 4 atomes de carbone et portant 1 à 4 atomes d'halogènes identiques ou différents ; amino ; alkylamino ou dialkylamino ayant 1 à 4 atomes de carbone.

**3.** Amides dérivés de cyclopropanoylamino-acides de formule (I) et suivant la revendication 1, formule dans laquelle

| | |
|---|---|
| $R^1$ , $R^2$, $R^3$, $R^4$ et $R^5$ | sont identiques ou différents et représentent l'hydrogène ou un groupe alkyle à chaîne droite ou ramifiée ayant 1 à 4 atomes de carbone, |
| Q | est une chaîne alkylénique de 1 à 4 atomes de carbone, droite ou ramifiée, non substituée ou portant un substituant phényle et/ou benzyle, les restes phényle et/ou benzyle étant, quant à eux, non substitués ou portant un à trois substituants, identiques ou différents, fluor, chlore, brome, cyano, nitro, méthyle, éthyle, n-propyle, isopropyle, n-butyle, isobutyle, sec.-butyle, tertio-butyle, méthoxy, éthoxy, n-propoxy, isopropoxy, trifluorométhyle et trifluorométhoxy ; ou bien Q forme conjointement avec le reste $R^4$ et l'atome d'azote un noyau de pyrrolidine, |
| A | est une chaîne alkylénique droite ou ramifiée ayant 1 à 4 atomes de carbone, |
| n | représente le nombre 0 ou 1 et |
| Ar | est un groupe phényle ou naphtyle non substitué ou portant un à trois substituants, identiques ou différents, en considérant comme substituants : le fluor, le chlore, le brome, un reste cyano, nitro, méthyle, éthyle, n-propyle, isopropyle, n-butyle, isobutyle, sec.-butyle, tertio-butyle, méthoxy, éthoxy, n-propoxy, isopropoxy, trifluorométhyle et trifluorométhoxy. |

**4.** Procédé de production d'amides dérivés de cyclopropanoylamino-acides de formule générale (I)

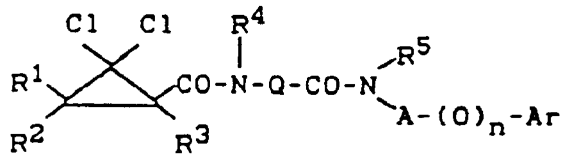

(I)

dans laquelle

| | |
|---|---|
| $R^1$, $R^2$, $R^3$, $R^4$ et $R^5$ | sont identiques ou différents et représentent l'hydrogène ou des restes alkyle ; |
| Q | représente une chaîne alkylénique droite ou ramifiée, non substituée ou substituée, ou forme conjointement avec le reste $R^4$ et l'atome d'azote un noyau de pyrrolidine ; |
| A | représente une chaîne alkylénique droite ou ramifiée ; |
| n | est le nombre 0 ou 1 et |
| Ar | et un groupe aryle non substitué ou substitué, |

caractérisé en ce que :

a) on fait réagir l'acide cyclopropanecarboxylique de formule (II)

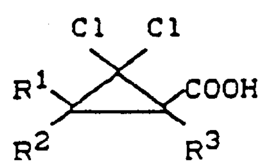

(II)

dans laquelle

$R^1$, $R^2$ et $R^3$ ont la définition indiquée ci-dessus ou leurs dérivés à groupe carboxy activé, avec

un amino-acide de formule (III)

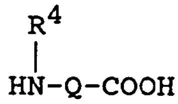 (III)

dans laquelle

$R^4$ et Q ont la définition indiquée ci-dessus, le cas"-échéant en présence d'un catalyseur, en la présence éventuelle d'un accepteur d'acide et, le cas échéant, en présence d'un diluant, puis on fait réagir, le cas échéant après conversion en une forme à groupe carboxy activé, le cyclopropanoylamino-acide ainsi obtenu de formule (IV)

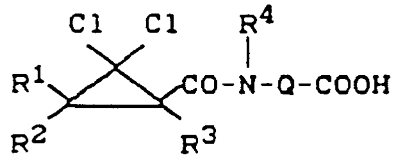 (IV)

dans laquelle

$R^1$, $R^2$, $R^3$, $R^4$ et Q ont la définition indiquée ci-dessus, avec une amine de formule (V)

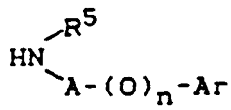 (V)

dans laquelle

$R^5$, A, n et Ar ont la définition indiquée ci-dessus, le cas échéant en présence d'un catalyseur, en la présence éventuelle d'un accepteur d'acide et, le cas échéant, en présence d'un diluant,

ou bien

b) on fait réagir un amino-acide, à fonction amino protégée par un groupe protecteur, de formule (VI)

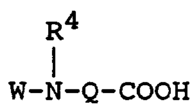 (VI)

dans laquelle

$R^4$ et Q ont la définition indiquée ci-dessus et W représente un groupe protégeant la fonction amino, ou leurs dérivés à groupe carboxy activé, le cas échéant en présence d'un catalyseur, en la présence éventuelle d'un accepteur d'acide et, le cas échéant, en présence d'un diluant, avec une amine de formule (V)

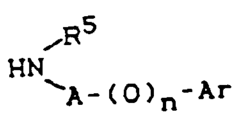 (V)

dans laquelle

$R^5$, A, n et Ar ont la définition indiquée ci-dessus, puis on fait réagir l'amide d'amino-acide ainsi obtenu de formule (VII)

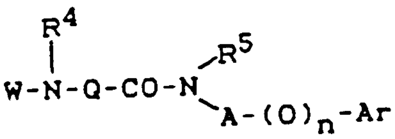

(VII)

dans laquelle

W, $R^4$, Q, $R^5$, A, n et Ar ont la définition indiquée ci-dessus, après élimination du groupe W protégeant la fonction amino, avec l'acide cyclopropanecarboxylique de formule (II)

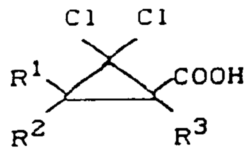

(II)

dans laquelle

$R^1$, $R^2$ et $R^3$ ont la définition indiquée ci-dessus, ou avec des dérivés à groupe carboxy activé de cet acide, le cas échéant en présence d'un catalyseur, en la présence éventuelle d'un accepteur d'acide et, le cas échéant, en présence d'un diluant.

5. Composition pesticide, caractérisée par une teneur en au moins un amide dérivé de cyclopropanoylaminoacide de formule (I) suivant les revendications 1 à 4.

6. Utilisation d'amides dérivés de cyclopropanoylamino-acides de formule (I) suivant les revendications 1 à 4 pour combattre des parasites.

7. Procédé pour combattre des parasites, caractérisé en ce qu'on fait agir des amides dérivés de cyclopropanoylamino-acides de formule (I) suivant les revendications 1 à 4 sur les parasites et/ou sur leur milieu vital.

8. Procédé de préparation de compositions pesticides, caractérisé en ce qu'on mélange des amides dérivés de cyclopropanoylamino-acides de formule (I) suivant les revendications 1 à 4 avec des diluants et/ou des agents tensio-actifs.

9. Dérivés de cyclopropanoylamino-acides de formule générale (IV)

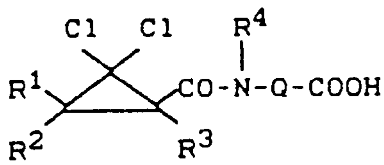

(IV)

dans laquelle

| | |
|---|---|
| $R^1$, $R^2$, $R^3$ et $R^4$ | sont identiques ou différents et représentent l'hydrogène ou des restes alkyle et |
| Q | est une chaîne alkylénique droite ou ramifiée non substituée ou substituée, ou forme conjointement avec le reste $R^4$ et l'atome d'azote un noyau de pyrrolidine, |

à l'exception du composé dans lequel $R^1$ et $R^2$ représentent un groupe méthyle, $R^3$ et $R^4$ représentent l'hydrogène et Q est un groupe $-CH_2-$.